(19) Europäisches Patentamt
European Patent Office
Office européen des brevets

(11) Numéro de publication : **0 618 218 A1**

(12) **DEMANDE DE BREVET EUROPEEN**

(21) Numéro de dépôt : **94400675.8**

(51) Int. Cl.$^5$ : **C07H 19/20**

(22) Date de dépôt : **29.03.94**

(30) Priorité : **31.03.93 FR 9303744**

(43) Date de publication de la demande : **05.10.94 Bulletin 94/40**

(84) Etats contractants désignés : **CH DE FR GB IT LI**

(71) Demandeur : **COMMISSARIAT A L'ENERGIE ATOMIQUE
31-33, rue de la Fédération
F-75015 Paris (FR)**

(72) Inventeur : **Lellouche, Jean-Paul
13, Résidence Tournemire
F-91940 Les Ulis (FR)**
Inventeur : **Levannier, Karine
1, Allée de la Cascade
F-92500 Rueil-Malmaison (FR)**
Inventeur : **Mioskowski, Charles
14, rue Baudelaire
F-67200 Strasbourg (FR)**

(74) Mandataire : **Des Termes, Monique et al
Société Brevatome
25, rue de Ponthieu
F-75008 Paris (FR)**

(54) **Procédé de synthèse de dérivés acylés de thiols transporteurs d'acides gras, en particulier d'acyl-coenzymes A, et les acyl-coenzymes A ainsi obtenus.**

(57) L'invention concerne un procédé de synthèse d'un dérivé acylé d'un thiol transporteur d'acides gras tel que le coenzyme A.

Selon ce procédé, on prépare un thiol silylé ou stannylé par réaction du thiol avec un réactif de silylation ou de stannylation pour remplacer au moins en partie les hydrogènes labiles du thiol par des groupes $SiR_3$ ou $SnR_3$ avec R étant un groupe alkyle ou aryle. On fait ensuite réagir le thiol silylé ou stannylé, en milieu organique anhydre, avec un acide organique activée et un réactif de déprotection capable d'éliminer les groupes silyle ou stannyle.

On peut ainsi obtenir des dérivés acylés du coenzyme A dans lesquels le groupe acyle peut avoir jusqu'à 30 atomes de carbone, avec des rendements élevés.

EP 0 618 218 A1

La présente invention a pour objet un procédé de synthèse de dérivés acylés de thiols transporteurs d'acides gras, en particulier d'acyl-coenzymes A.

Les acyl-coenzymes A sont des dérivés très intéressants qui peuvent être utilisés dans de nombreuses voies biosynthétiques, en particulier pour l'étude des mécanismes biologiques impliquant des acides gras.

On connait plusieurs méthodes de préparation des acyl-coenzymes A. Ces méthodes utilisent pour la plupart la réaction de condensation d'un acide activé avec le coenzyme A, à la température ambiante, en phase aqueuse ou dans un milieu eau-solvant organique. L'acide activé peut être un anhydride mixte, un chlorure, ou un ester de N-hydroxysuccinimide ou un acyl imidazole, comme il est décrit par E.R.Staddtman, Methods in Enzymol. 1957, Vol 3, 931-941 ; Peter Goldman et P. Roy Vagelos, J. Biol. Chem. 1961, 236, 2620-2623 ; Frank Davidoff and Edward D. Korn, J. Biol. Chem. 1964, 239, 2496-2506 ; Adhid Al-Arif et Melvin Blecher, J. Lipid Res. 1969, 10, 344-345 ; Adhid Al-Arif et Melvin Blecher, Biochim. Biophys. Acta. 1971, 248, 416-429 ; Maynard E. Pullman, Anal. Biochem. 1973, 54, 188-198 ; James E. Bishop et Amiya K. Hajra, Anal. Biochem. 1980, 106, 344-350 ; et Akihiko Kawaguchi, Tsutomu Yoshimura et Shigenobu Okuda, J. Biochem. 1981, 89, 337-339.

Ces méthodes sont difficiles à mettre en oeuvre car elles sont basées sur la réaction d'un composé très hydrophile (coenzyme A) qui est soluble dans l'eau mais insoluble dans la plupart des solvants organiques, avec un acide organique qui est souvent insoluble dans l'eau. De ce fait, pour réaliser la réaction de condensation, il est nécessaire de mélanger une solution aqueuse dans laquelle est dissous le coenzyme A avec un solvant organique contenant l'acide activé et de contrôler le pH tout au long de la réaction, ce qui donne lieu à des difficultés de mise en oeuvre. De plus, cette méthode ne peut être utilisée lorsque l'acide activé a une très longue chaîne hydrocarbonée, par exemple un nombre d'atomes de carbone supérieur à 18. Enfin, les rendements obtenus par ces méthodes ne sont pas reproductibles en raison des difficultés de mise en oeuvre de la réaction.

La présente invention a précisément pour objet un procédé de synthèse de dérivés acylés de thiols transporteurs d'acides gras, qui pallie ces inconvénients et s'applique de plus à la préparation de dérivés acylés dans lesquels le radical acyle a plus de 18 atomes de carbone.

Selon l'invention, le procédé de synthèse d'un dérivé acylé d'un thiol transporteur d'acides gras par réaction du thiol avec un réactif d'acylation, comprend les étapes suivantes :

1°) préparer un thiol silylé ou stannylé en faisant réagir dans un solvant organique le thiol avec un réactif de silylation ou de stannylation pour remplacer au moins en partie les hydrogènes labiles du thiol par des groupes de formule $SiR_3$ ou $SnR_3$ dans lesquelles les R qui peuvent être identiques ou différents, sont des groupes alkyle ou aryle, et

2°) faire réagir dans un solvant organique anhydre le thiol silylé ou stannylé avec a) un réactif de déprotection pour éliminer les groupes $SiR_3$ ou $SnR_3$ et avec b) le réactif d'acylation pour obtenir le dérivé acylé du thiol.

Dans ce procédé, le fait de préparer dans la première étape un thiol silylé ou stannylé permet de solubiliser le thiol transporteur d'acides gras qui présente généralement un fort caractère hydrophile, dans un solvant organique anhydre et de faciliter ensuite la réaction de condensation du thiol avec le réactif d'acylation.

De ce fait, on peut réaliser ensuite cette réaction de condensation dans un milieu organique anhydre, sans avoir à contrôler le pH, ce qui permet d'obtenir des rendements élevés en dérivés acylés de façon reproductible.

Les thiols transporteurs d'acides gras utilisés dans le procédé de l'invention sont des molécules organiques plus ou moins complexes, comportant un groupe SH par lequel elles peuvent être associées à divers acides gras.

A titre d'exemple de telles molécules, on peut citer le coenzyme A qui est une molécule complexe comportant de nombreux hydrogènes labiles, la cystéamine qui est une molécule beaucoup plus simple de formule $NH_2$-$C_2H_4$-SH, la carnitine et l'acide panthénoïque.

La formule du coenzyme A est la suivante :

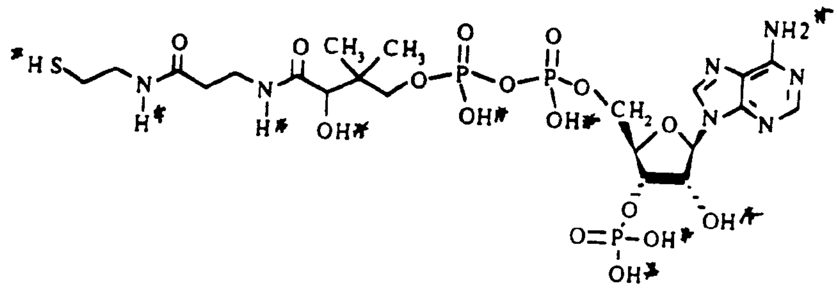

D'après cette formule, on voit qu'il est possible de protéger les 11 fonctions (hydroxyle, amide, amine phosphate et thiol) indiquées ci-dessus par des astérisques, par des groupes silyle ou stannyle.

Le remplacement d'hydrogènes labiles par de tels groupes, dans ce composé hydrophile, très polaire, réduit cette polarité, diminue ainsi les possibilités de formation de liaisons hydrogène et favorise donc ,de façon générale, la solubilité du composé dans des solvants organiques polaires et aussi non polaires.

Aussi, le procédé de l'invention est particulièrement intéressant pour préparer les dérivés acylés du coenzyme A.

Dans la première étape du procédé de l'invention, on prépare donc un thiol silylé ou stannylé en faisant réagir, dans un solvant organique le thiol de départ avec un réactif de silylation ou de stannylation afin de remplacer au moins en partie les hydrogènes labiles du thiol par des groupes de formules $SiR_3$ ou $SnR_3$ dans lesquelles les R qui peuvent être identiques ou différents, sont des groupes alkyle ou aryle.

Les groupes alkyle peuvent être linéaires ou ramifiés et ils ont généralement de 1 à 6 atomes de carbone, de préférence 1 à 4 atomes de carbone. Les groupes aryle ont généralement de 6 à 15 atomes de carbone.

A titre d'exemple de tels groupes aryle, on peut citer phényle et naphtyle.

De préférence, dans la première étape, on fait réagir le thiol avec un réactif de silylation.

On peut utiliser divers réactifs de silylation, mais on préfère en particulier utiliser le 1-méthoxy-2-méthyl-1-triméthylsiloxypropène car en faisant réagir le thiol avec ce réactif, on obtient comme sous-produit de la réaction des composés très volatils, acétate d'isopropyle et hexaméthyldisiloxane ayant respectivement des points d'ébullition de 85°C et 101°C, qui peuvent donc être éliminés facilement du milieu réactionnel par évaporation.

Par ailleurs, lorsque le thiol est le coenzyme A, l'utilisation de ce réactif de silylation présente l'avantage de silyler les fonctions thiols en milieu neutre, ce qui évite la dimérisation du coenzyme A.

On peut effectuer la réaction de silylation directement sur le thiol ou en le mettant en solution dans un solvant organique tel que l'acétonitrile ou le dichlorométhane, et en ajoutant à la solution l'agent de silylation que l'on laisse réagir à la température ambiante pendant la durée voulue. En fin de réaction, on élimine le solvant si nécessaire, les sous-produits de la silylation et l'excès de réactif par évaporation sous pression réduite.

Dans le cas du coenzyme A, la réaction de silylation peut conduire à une protection des 11 fonctions indiquées précédemment. Toutefois, dans les conditions réactionnelles utilisées, il n'est pas certain que toutes ces fonctions soient silylées, mais on obtient habituellement la silylation de 7 fonctions dont la fonction thiol.

Dans le cas où on réalise une réaction de stannylation, on peut utiliser comme réactif de stannylation, un réactif analogue et opérer dans des conditions similaires.

Après silylation ou stannylation, on effectue la réaction de condensation du thiol sur le réactif d'acylation, en milieu organique anhydre, en utilisant de plus un réactif de déprotection pour éliminer les groupes $SiR_3$ ou $SnR_3$ sur les fonctions thiols qui doivent réagir avec le réactif d'acylation.

Pour effectuer cette déprotection, on peut utiliser comme réactif un fluorure ou d'autres réactifs capables d'éliminer les groupes $SiR_3$ ou $SnR_3$.

A titre d'exemples de tels réactifs de déprotection, on peut citer le fluorure de tétrabutylammonium, le difluorotriphénylstannate de tétrabutylammonium, le trichlorure d'aluminium, le fluorure de potassium, le fluorure de césium et le fluorure de rubidium.

De préférence, selon l'invention, on utilise comme réactif de déprotection le fluorure de césium ou le fluorure de rubidium.

En effet, les autres fluorures donnent des résultats moins bons et il en est de même des autres réactifs mentionnés ci-dessus.

Avec le fluorure de césium et le fluorure de rubidium, on peut de plus accélérer la réaction, soit en soumettant le milieu réactionnel à une sonication, soit en opérant en présence d'un catalyseur constitué par un éther-couronne tel que le dicyclohexyl-18-C-6 (DCH-18-C-6) de formule :

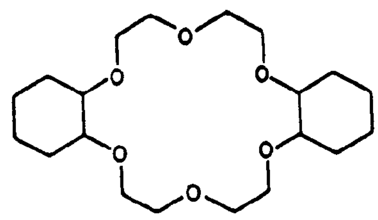

ou la tris (3,6-dioxaheptyl)amine (TDA-1) de formule :

$N(CH_2CH_2OCH_2CH_2OCH_3)_3$.

L'utilisation de TDA-1 est avantageuse, car ce produit n'est pas toxique.

Pour effectuer la condensation, on peut utiliser comme réactif d'acylation, les acides activés, saturés ou insaturés, généralement utilisés tels que les anhydres mixtes, les chlorures d'acide, les thioesters et les esters.

Ces acides activés peuvent être substitués par divers substituants tels que OH, F, le groupe cyclopropyle etc... Des exemples d'acides activés de ce type sont décrits dans Journal of American Chemical Society, vol. 114, 1992, p. 2245-2251 ; vol. 113, 1991, p. 7388-7397 et vol. 115, 1993, p. 1619-1628.

De préférence, selon l'invention, on utilise comme réactif d'acylation, un ester ou un anhydride mixte d'un acide carboxylique saturé ou insaturé, éventuellement substitué.

L'ester peut être en particulier un ester d'un acide carboxylique et du N-hydroxysuccinimide, du N-hydroxyphtalimide ou de l'alcool 2,4,6-trichlorobenzylique.

A titre d'exemples d'acides carboxyliques utilisables, on peut citer l'acide octadécanoïque, l'acide icosanoïque, l'acide arachidonique, l'acide oléique, l'acide linoléique, l'acide linolénique l'acide 3-hydroxy-icosanoïque, l'acide 3-oxo-icosanoïque, l'acide 2-icosènoïque, l'acide (2-heptadécyl-1,3-dioxolan-2-yl)-acétique, l'acide 2-fluoro-2-ico-sénoïque et l'acide 4-fluoro-3-hydroxy-icosanoïque.

Les solvants organiques utilisés dans cette deuxième étape pour effectuer la réaction de désilylation ou de déstannylation et de condensation, peuvent être choisis parmi les solvants organiques polaires et non polaires tels que le tétrahydrofurane (THF), le dichlorométhane, l'hexane, l'acétonitrile, l'acétone, l'éther diméthylique, l'acétate d'éthyle, l'alcool, l'éther diéthylique.

De préférence, dans l'invention, on utilise comme solvant organique l'éther diméthylique, le tétrahydrofurane, l'acétonitrile et leurs mélanges.

Dans la deuxième étape, on choisit, par ailleurs, les quantités de réactif d'acylation et de thiol utilisées de façon à optimiser le rendement en dérivé acylé. De bons résultats sont obtenus lorsque le rapport molaire du réactif d'acylation au thiol est de 1:1 à 2:1.

On peut effectuer cette deuxième étape à la température ambiante ou à une température inférieure ou même supérieure, par exemple à des températures de 0 à 25°C, pendant des durées allant de 1h à 10h.

La température et la durée de la réaction de condensation dépendent en particulier du réactif de déprotection utilisé.

Ainsi, lorsqu'on utilise comme réactif le fluorure de césium, en présence de DCH-18-C-6, on préfère opérer à une température de 25°C pendant 3 à 5h.

En revanche, lorsqu'on utilise comme réactif de déprotection, le fluorure de césium, en soumettant le milieu réactionnel à une sonication, on préfère opérer à 0°C pendant environ 1h30.

L'invention a aussi pour objet le coenzyme A silylé ou stannylé obtenu dans la première étape du procédé. Ce coenzyme A silylé répond à la formule suivante :

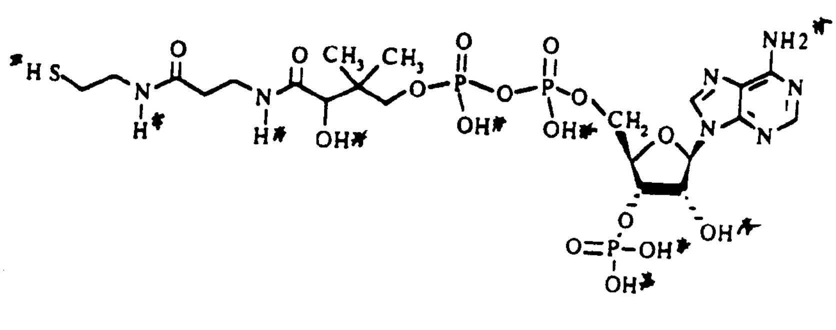

dans laquelle au moins une partie des hydrogènes labiles H* sont remplacés par un groupe de formule $SiR_3$ dans laquelle les R qui peuvent être identiques ou différents sont des groupes alkyle, en particulier des grou-

pes alkyle en $C_1$ à $C_4$, ou des groupes aryle.

L'invention a encore pour objet les acyl-coenzymes A obtenus par ce procédé dans lesquels le groupe acyle répond à la formule $R^1CO$- dans laquelle $R^1$ est un groupe hydrocarboné comprenant de 20 à 29 atomes de carbone et de 0 à 6 doubles liaisons.

D'autres caractéristiques et avantages de l'invention apparaîtront mieux à la lecture des exemples qui suivent, donnés bien entendu à titre illustratif et non limitatif.

Ces exemples illustrent la préparation de dérivés acylés du coenzyme A (CoASH), obtenus par réaction du coenzyme A silylé (CoASH silylé) avec un acide activé (acide icosanoïque ou acide octadécanoïque de formule $R^1$-COOH avec $R^1$ représentant $CH_3(CH_2)_{16}$ ou $CH_3(CH_2)_{18}$) conformément au procédé de l'invention.

Cette préparation correspond au schéma réactionnel donné ci-dessous qui se rapporte aux exemples 1 et 2.

1ère étape

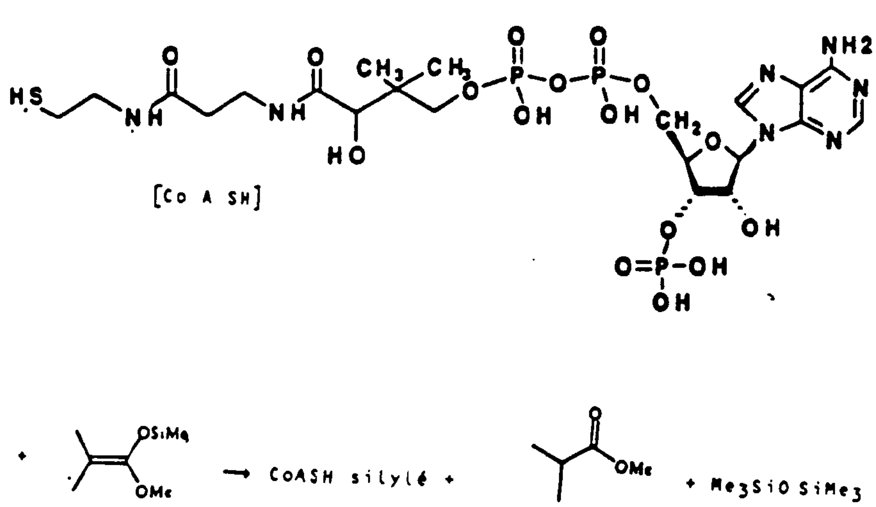


2ième étape

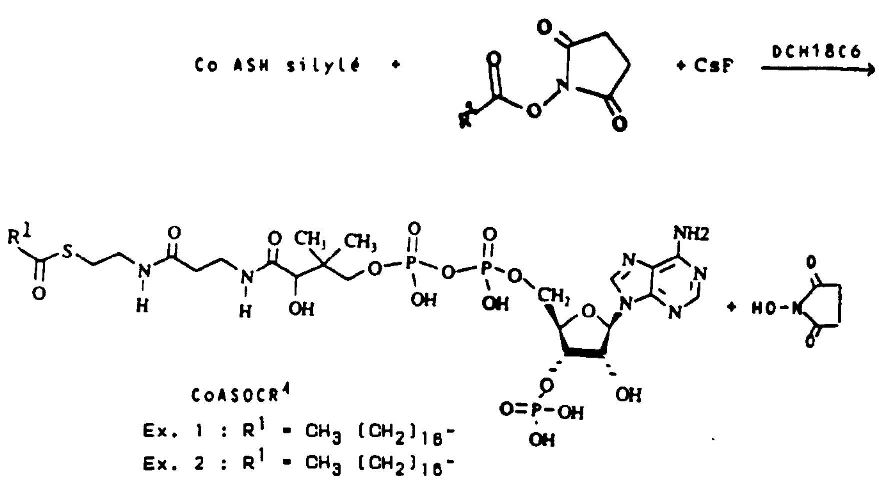


**Exemple 1** : Préparation de l'icosanoyl coenzyme A.

1) Silylation du coenzyme A.

Dans un flacon vial de 1ml contenant lOmg (13, Oμ mol) de coenzyme A, trihydrate (Sigma) en solution dans 0,5ml d'acétonitrile fraîchement distillé, on ajoute 30 μl (104,0μmol) de 1-méthoxy-2-méthyl-1-triméthylsilyloxypropène (Aldrich). On soumet l'ensemble à une agitation à 25°C sous azote et on constate que le milieu

devient homogène après 40min de réaction. Après 12h de réaction, on évapore le solvant, les sous-produits de la silylation et l'excès de réactif sous pression réduite pour obtenir une huile incolore constituée par le dérivé silylé du coenzyme A.

2) Préparation du dérivé acylé du coenzyme A.

a) Préparation de l'ester de l'acide icosanoïque et du N-hydroxysuccinimide.

A 3,0mmol d'acide icosanoïque en solution dans 10ml d'acétate d'éthyle anhydre, on ajoute 340mg (3,0mmol) de N-hydroxysuccinimide (Aldrich) en solution dans 3ml d'acétate d'éthyle anhydre, puis on ajoute 610mg (3,0mmol) de dicyclohexyl carbodiimide (Aldrich) en solution dans 3ml d'acétate d'éthyle anhydre. On maintient le mélange réactionnel sous azote à la température ambiante pendant une nuit, puis on le filtre pour éliminer la dicyclohexyl urée formée et on le soumet à une concentration sous vide. On purifie ensuite le produit par chromatographie sur gel de silice en utilisant pour l'élution un mélange éther/éther de pétrole (50/50), puis on recristallise dans l'éthanol, et on obtient une poudre blanche (rendement de 95%).

Les caractéristiques de ce produit sont les suivantes :

- spectrométrie de masse : (DCI/$NH_3$) M + 18 = 427
- analyse élémentaire : (en %)
  calculé : C 70,37, H 10,58, O 16,6, N 3,42
  trouvé : C 70,09, H 10,3, O 15,75, N 3,31.
- IR ($cm^{-1}$) : 2990 ( υ C-H), 1820, 1740 ( υ C=O), 1450 (δ CH2), 1375 (δCH3), 1200( υ C-O), 1060
- RMN$^1$H ($CDCl_3$ 300 MHz) : δ 0,86 (t, J=6,3 Hz, 3 H, $CH_3$), δ 1,24 (28 H, $CH_2$), δ 1,78 (tt, 2 H, $\underline{CH_2}$-$CH_2$-$CO_2N$), δ 2,58 (t, J=6,2 Hz, 2 H, $CH_2$-$\underline{CH_2}$-$CO_2N$)δ 2,82 (s, 4H, N-CO-$\underline{CH_2}$-$\underline{CH_2}$-CO-N).
- RMN $^{13}C$ ($CDCl_3$ 75MHz) : δ 168,41 (O-$\underline{C}$=O), δ 168,86 (N-$\underline{C}$=O).

b) Condensation du coenzyme A avec l'ester de N-hydroxysuccinimide.

Dans un ballon de 25ml contenant 50mg (25 équiv.) de fluorure de césium, 15mg (10% en mol par rapport au fluorure de césium) de dicyclohexyl-18-C-6 et 7,5mg (1,5équiv.) de l'ester d'acide icosanoïque et de N-hydroxysuccinimide obtenu précédemment, on ajoute 13μ mol du coenzyme A silylé obtenu dans la première étape en solution dans 1ml de THF fraîchement distillé. On maintient le milieu réactionnel sous azote pendant 6h à 0°C, puis on arrête la réaction par addition de $KH_2PO_4$ 10mM, on filtre sur filtre Anotop $10^+$ (0,2μm) et on analyse le produit obtenu par chromatographie liquide à haute performance (HPLC) analytique.

Pour la chromatographie analytique, on utilise une colonne analytique, Nucléosil C-18 5μm (250 x 4,6mm, Interchim), dans les conditions suivantes:

- AU = 0,01
- $\lambda_{max}$= 254nm,
- vitesse d'élution = 1,5ml/min
- éluant : mélange de A ($KH_2PO_4$10mM) et de B ($CH_3CN$) avec un gradient linéaire dans les conditions suivantes : on passe en 10min de 60% A-40% B à 55% A-45%B, puis en 10min à 45% A - 55% B, en 15min à 15% A-85% B et en 10min à 60% A - 40% B.

Pour la chromatographie semipréparative, on utilise une colonne Nucléosil C18 5μm (250 x 10mm, Interchim) dans les conditions suivantes :

- AU=0,1
- $\lambda_{max}$=254nm,
- vitesse d'élution = 1,5ml/min
- éluant : mélange de A ($KH_2PO_4$10mM) et de B ($CH_3CN$) avec un gradient linéaire dans les conditions suivantes : on passe en 10min de 60% A - 40% B à 55% A - 45% B, puis en 10min à 45% A - 55% B, en 15min à 20% A - 80% B et en 10min à 60% A - 40% B.

Les temps de rétention des différents produits caractérisés sont de 1min pour le CoASH, 1,2min pour le dimère $(CoAS)_2$, et 23min pour l'icosanoyl-CoA.

L'acétonitrile est évaporé sous pression réduite et la phase aqueuse restante est lyophilisée. Le produit est ensuite dessalé sur la même colonne après qu'elle ait été rincée à l'eau. Le résidu est déposé sur la colonne, lavé à l'eau pendant 40min avec une vitesse d'élution de 1,5ml/min, puis élué au méthanol à la même vitesse. Le solvant est ensuite concentré et le produit obtenu est resolubilisé dans l'eau, puis lyophilisé et conservé à -18°C.

Les caractéristiques du produit obtenu sont données dans le tableau 1 annexé.

**Exemple 2** : Préparation de l'octadécanoyl-coenzyme A.

On suit le même mode opératoire que dans l'exemple 1 pour préparer ce dérivé acylé, sauf que l'on prépare l'ester du N-hydroxysuccinimide en utilisant l'acide octadécanoïque au lieu de l'acide icosanoïque.

Les caractéristiques du produit obtenu sont données dans le tableau 1 annexé.

**Tableau 1**

| | **Ex. 1 : icosanoyl coenzyme A** | **Ex. 2 : octadécanoyl coenzyme A** |
|---|---|---|
| **Rendement : %** | 78 | 78 |
| **RMN$^1$H (300 MHz) :** | **RMN $^1$H ($CDCl_3$ 300 MHz) :** δ 0,86 (t, *J* = 6,3 Hz, 3 H, $CH_3$), δ 1,24 (28 H, $CH_2$), δ 1,55(tt, 2 H, $\underline{CH_2}$-$CH_2$-$C0_2S$), δ 2,9(t, *J* = 6,2 Hz, 2 H, $CH_2$-$\underline{CH_2}$-$C0_2S$) | |
| **UV : nm** | ε 257 nm = 7809 | ε 257 nm = 12509 |
| | ε 197 nm =10236 | ε 197 nm = 25835 |
| **FAB : m/z** | 1063 | |
| **Rf : min** | 23 | 18 |

**Exemple 3:** Préparation de l'icosanoyl-coenzyme A.

Dans cet exemple, on suit le même mode opératoire que dans l'exemple 1 pour préparer le dérivé silylé du coenzyme A, et on réalise ensuite l'étape de condensation et de déprotection de la façon suivante en utilisant comme acide activé l'ester de l'acide icosanoïque et du N-hydroxysuccinimide préparé de la même façon que dans l'exemple 1.

Dans un ballon de 25ml contenant 50mg de fluorure de césium (25équiv.), 15mg (10% en mol) de DCH-18-C-6, 7,5mg (1,5équiv.) d'acide activé au N-hydroxysuccinimide, on ajoute léquiv. du coenzyme A silylé en solution dans 1ml de $THF/CH_3CN$ (2/1). On maintient le milieu réactionnel sous azote pendant 6h à 25°C. On arrête alors la réaction par addition de $KH_2PO_4$ 10mM, puis on sépare le produit obtenu, on le purifie et on l'analyse dans les mêmes conditions que celles de l'exemple 1.

Le rendement de la réaction est de 75%.

**Exemples 4 à 6** : Préparation de l'icosanoyl-coenzyme A.

Dans ces exemples, on suit le même mode opératoire que dans l'exemple 3, sauf en ce qui concerne la quantité d'acide activé utilisée de façon à faire varier le rapport acide activé/coenzyme A de 2/1 à 1/2.

Les rapports utilisés et les rendements obtenu dans ces conditions sont donnés dans le tableau 2 ci-dessous.

**TABLEAU 2**

| EX. | RAPPORT MOLAIRE ACIDE ACTIVE/COENZYME A | RENDEMENT (%) (+ 5 %) |
|---|---|---|
| 3 | 1,5/1 | 75 |
| 4 | 2/1 | 75 |
| 5 | 1/1 | 69 |
| 6 | 1/2 | 60 |

**Exemples 7 à 18** : Préparation de l'icosanoyl-coenzyme A.

Dans ces exemples, on suit le même mode opératoire que dans l'exemple 3 pour préparer ce dérivé acylé du coenzyme A, mais on utilise des solvants organiques différents dans la deuxième étape de condensation. Les solvants utilisés et les rendements obtenus sont donnés dans le tableau 3.

**TABLEAU 3**

| EX | SOLVANTS | RENDEMENT (%) (± 5 %) |
|---|---|---|
| 7 | THF | 78,5 |
| 8 | $CH_3CN$ | 72 |
| | $CH_3CN/THF$ | |
| 9 | 2/1 | 65 |
| 10 | 1/1 | 75 |
| 11 | 1/2 | 75 |
| 12 | DME | 70 |
| 13 | $CH_2Cl_2$ | 30 |
| 14 | AcOEt | 50 |
| 15 | $Et_2O$ | 65 |
| 16 | acétone | 50 |
| 17 | EtOH | 30 |
| 18 | hexane | 10 |

**Exemple 19** : Préparation de l'icosanoyl-coenzyme A.

Dans cet exemple, on suit le même mode opératoire que dans l'exemple 1 pour préparer ce dérivé acylé, sauf en ce qui concerne la deuxième étape qui est réalisée dans les conditions suivantes.

On dissout 1équiv. du dérivé silylé obtenu dans la première étape de l'exemple 1 dans lml de THF anhydre auquel on ajoute 130µl de fluorure de tétrabutylammonium (8équiv.), puis on laisse sous agitation et sous azote pendant 5 min à 25°C. On ajoute alors 1,5équiv. de l'ester de la N-hydroxy succinimide de l'acide icosanoïque en solution dans 1ml de THF anhydre. Après 1h d'agitation à la température ambiante, on sépare le produit obtenu puis on l'analyse et on le dose par chromatographie liquide à haute performance dans les conditions décrites dans l'exemple 1.

Le rendement obtenu dans ces conditions est de 55%.

**Exemples 20 à 25** : Préparation d'icosanoyl-coenzyme A.

Dans ces exemples, on suit le même mode opératoire que dans l'exemple 19, sauf que l'on utilise d'autres solvants organiques.

Les résultats obtenus ainsi que les solvants utilisés sont donnés dans le tableau 4 ci-dessous.

**TABLEAU 4**

| EX | SOLVANTS | RENDEMENT (%) (± 5 %) |
|---|---|---|
| 19 | THF | 55 |
| 20 | $CH_3CN$ | 30 |
| 21 | DME | 10 |
| 22 | $CH_2Cl_2$ | 0 |
| 23 | AcOEt | 0 |
| 24 | $Et_2O$ | 40 |
| 25 | acétone | 60 |

**Exemple 26** : Préparation de l'icosanoyl-coenzyme A.

Dans cet exemple, on suit le même mode opératoire que dans l'exemple 1, sauf que l'on réalise la deuxième étape de condensation dans les conditions suivantes.

Dans un ballon de 25ml contenant 50équiv. de fluorure de potassium, 10% en mol (par rapport au fluorure de potassium) de DCH-18-C-6 et 1,5équiv. d'ester de N-hydroxysuccinimide, on ajoute léquiv. de coenzyme A silylé en solution dans 1ml du mélange THF/$CH_3CN$ (2/1). On maintient le milieu réactionnel sous azote pendant 3h à une température de 25°C.

On sépare ensuite le produit obtenu et on l'analyse par HPLC. Le rendement de la réaction est de 35/40%.

**Exemples 27 à 34** : Préparation de l'icosanoyl-coenzyme A.

Dans ces exemples, on suit le même mode opératoire que dans l'exemple 26, sauf que l'on utilise différents réactifs de déprotection, et éventuellement des quantités de réactif, des durées et des températures de réaction différentes.

Dans les exemples 31 et 32, on utilise comme réactif de déprotection du fluorure de césium seul, sans catalyseur à base d'éther-couronne, mais on soumet le mélange réactionnel à une sonication .

Les réactifs utilisés, leur quantité, la durée et la température de réaction ainsi que les rendements obtenus sont donnés dans le tableau 5.

**TABLEAU 5**

| EX | CATALYSEUR | durée (h) | T (°C) | rendement (%) (+ 5 %) |
|---|---|---|---|---|
| 26 | KF/dicyclohexyl-18-C-6 50éq./10 % M | 3 | 25 | 35/40 |
| 27 | CsF/dicyclohexyl-18-C-6 50éq/2 % M | 3 | 25 | 70 |
| 28 | CsF/dicyclohexyl-18-C-6 50éq/10 % M | 3 | 25 | 78 |
| 29 | CsF/dicyclohexyl-18-C-6 25éq/10 % M | 3 | 25 | 78 |
| 30 | CsF 25 éq. | 3 | 25 | 62 |
| 31 | CsF/sonication 25 éq. | 3 | 25 | 60 |
| 32 | CsF/sonication 25 éq. | 1h30 | 0 | 75 |
| 33 | $AlCl_3$ | | | 5 |
| 34 | $[\Phi_3SnF_2]^-[NBu_4]^+$ | | | 0 |

Au vu des résultats du tableau 5, on remarque que les meilleurs résultats sont obtenus lorsqu'on utilise le fluorure de césium comme réactif de déprotection, soit avec un éther-couronne, soit en soumettant le mélange à une sonication.

**Exemples 35 à 42** : Préparation de l'icosanoyl coenzyme A.

Dans ces exemples, on suit le même mode opératoire que dans l'exemple 3 mais on utilise pour effectuer la deuxième étape de condensation des températures et des durées de réaction différentes.

Les températures, les durées de réaction et les rendements obtenus sont donnés dans le tableau 6.

**TABLEAU 6**

| EX. | TEMPERATURE (°C) | TEMPS DE REACTION (h) | RENDEMENT (%) (+ 5 %) |
|---|---|---|---|
| 35 | -10 | 6 | <30 |
| 36 | 0 | 6 | 78 |
| 37 | 25 | 3 | 72 |
| 38 | 40 | 1h30min | 60 |
| 39 | 60 | <30min | <30 |
| 40 | 25 | 1h30min | 40 |
| 41 | 25 | 4h30min | 75 |
| 42 | 25 | 24 | 70 |

**Exemple 43:** Préparation de l'icosanoyl-coenzyme A.

Dans cet exemple, on suit le même mode opératoire que dans l'exemple 3 pour préparer le dérivé silylé du coenzyme A, puis on réalise la deuxième étape en utilisant à la place de l'ester de N-hydroxysuccinimide, l'acide icosanoïque activé par le chloroformate d'isobutyle . Pour préparer cet acide activé, on opère de la façon suivante.

A 26,0μmol d'acide icosanoïque en solution dans 0,5ml de dichlorométhane anhydre, on ajoute 26,0μmol (3,8μl) de triéthylamine anhydre sous argon.

Après 10min sous agitation, on ajoute au mélange réactionnel 26,0 μmol (3,5μl) de chloroformate d'isobutyle (Aldrich) à 0°C, puis on laisse le mélange réactionnel à la température ambiante pendant 2h. On obtient ainsi une suspension blanche que l'on filtre sous azote et que l'on concentre sous pression réduite pour obtenir un solide blanc.

On réalise ensuite la condensation de cet acide activé sur le coenzyme A en opérant dans les mêmes conditions que celles de l'exemple 3, c'est-à-dire en utilisant 50 équiv. de fluorure de césium en présence de DCH-18-C-6 (10% en mol), un rapport molaire acide activé / coenzyme A de 1,5/1 et une durée de réaction de 6h à 25°C.

Le rendement obtenu dans ces conditions est de 50%.

**Exemple 44.**

Dans cet exemple, on suit le même mode opératoire que dans l'exemple 43, sauf que l'on utilise comme acide activé l'acide activé par le chlorure de 2,4,6-trichlorobenzoyle, en préparant cet acide activé de la façon suivante.

A 19,0μmol d'acide icosanoïque en solution dans 2ml de THF anhydre, on ajoute 19,5μmol (27μl) de triéthylamine anhydre sous argon. On refroidit le milieu réactionnel dans un bain de glace et on y ajoute goutte à goutte à 0°C 19,5μmol (30,0μl) de chlorure de 2,4,6-trichlorobenzoyle. On maintient le milieu réactionnel à la température ambiante pendant 2h et on obtient ainsi une suspension blanche que l'on filtre sous azote et que l'on concentre sous pression réduite pour obtenir l'acide activé sous forme de solide blanc.

On effectue ensuite l'étape de condensation dans les mêmes conditions que celles de l'exemple 43.

On obtient ainsi un rendement en icosanoylcoenzyme A de 65%

Les exemples donnés ci-dessus montrent que l'on peut obtenir les acyl-coenzymes A avec de bons rendements, en particulier lorsqu'on utilise comme acide activé un ester de la N-hydroxysuccinimide et que l'on réalise la deuxième étape de condensation dans un solvant organique tel que THF, le mélange acétonitrile - THF (1/2) et l'éther diméthylique, en utilisant le fluorure de césium comme réactif de déprotection, soit à 0°C pendant 1h30min avec sonication, soit à 25°C pendant 3 à 5h en présence de DCH-18-C-6.

Par ailleurs,bien que dans ces exemples, on ait utilisé des acides saturés, l'invention s'applique également à l'emploi d'acides activés insaturés comprenant par exemple de 1 à 6 doubles liaisons.

**EXEMPLE 45 :** Préparation de l'octadécanoyl-coenzyme A.

On suit le même mode opératoire que dans l'Exemple 2 pour préparer ce dérivé acylé du coenzyme A sauf que l'on effectue la réaction pendant 5 heures. Le réactif d'acylation, les conditions réactionnelles, le produit obtenu et le rendement obtenu dans ces conditions sont donnés dans le tableau 7 qui suit.

**EXEMPLE 46 :** Préparation de l'octadécanoyl-coenzyme A.

On suit le même mode opératoire que dans l'Exemple 45, sauf que l'on utilise comme réactif d'acylation l'ester de l'acide octadécanoïque et du N-hydroxyphtalimide et que l'on réalise la réaction en utilisant comme réactif de déprotection le fluorure de rubidium au lieu du fluorure de césium.

Le réactif d'acylation, les conditions réactionnelles, le dérivé obtenu ainsi que le rendement en produit isolé sont donnés dans le tableau 7. Au vu de ce tableau, on remarque que le remplacement du fluorure de césium par le fluorure de rubidium et l'utilisation de l'ester du N-hydroxyphtalimide permet d'obtenir un rendement amélioré en produit.

**EXEMPLE 47 :** Préparation de l'icosanoyl-coenzyme A.

Dans cet exemple, on suit le même mode opératoire que dans l'Exemple 1 pour préparer ce dérivé acylé, mais on effectue la réaction pendant 5 heures.

Le réactif d'acylation, les conditions réactionnelles et le résultat obtenu sont donnés dans le tableau 7.

**EXEMPLE 48 :** Préparation de l'icosanoyl-coenzyme A.

Dans cet exemple, on suit le même mode opératoire que dans l'Exemple 47, mais on utilise comme catalyseur l'amine TDA-1 au lieu de l'éther-couronne.

Le réactif d'acylation, les conditions réactionnelles et le résultat obtenu sont donnés dans le Tableau 7.

**EXEMPLE 49 :** Préparation de l'icosanoyl-coenzyme A.

Dans cet exemple, on suit le même mode opératoire que dans l'Exemple 47, mais on utilise le fluorure de rubidium au lieu du fluorure de césium.

Le réactif d'acylation, les conditions réactionnelles et le résultat obtenu sont donnés dans le tableau 7.

**EXEMPLE 50** : Préparation de l'icosanoyl-coenzyme A.

Dans cet exemple, on suit le même mode opératoire que dans l'Exemple 49, mais le réactif d'acylation est constitué par l'ester du N-hydroxyphtalimide et le catalyseur est constitué par une amine le TDA-1.

Le réactif d'acylation, les conditions réactionnelles et le résultat obtenu sont donnés dans le tableau 7.

Si l'on compare les résultats obtenus dans les exemples 47 à 50, on remarque que les rendements en produit sont les mêmes quel que soit le réactif de déprotection, le catalyseur et le réactif d'acylation utilisés.

**EXEMPLE 51 :** Préparation du 2-fluoro-2-icosénoyl-coenzyme A.

Dans cet exemple, on suit le même mode opératoire que dans l'Exemple 45 pour préparer ce composé en utilisant comme réactif d'acylation l'ester de l'acide 2-fluoro-2-icosénoïque et du N-hydroxysuccinimide.

Le réactif d'acylation, les conditions réactionnelles et le résultat obtenu sont donnés dans le tableau 7.

**EXEMPLE 52** : Préparation du 2-fluoro-2-icosénoyl-coenzyme A.

Dans cet exemple, on suit le même mode opératoire que dans l'Exemple 51, mais on utilise comme réactif d'acylation l'ester de l'acide 2-fluoro-2-icosénoïque et du N-hydroxyphtalimide et comme réactif de déprotection le fluorure de rubidium au lieu du fluorure de césium.

Le réactif d'acylation, les conditions réactionnelles et le résultat obtenu sont donnés dans le tableau 7.

En comparant les résultats des Exemples 51 et 52, on remarque que l'utilisation de l'ester du N-hydroxyphtalimide et du fluorure de rubidium permet d'obtenir un rendement amélioré.

**EXEMPLE 53 :** Préparation du 4-fluoro-3-hydroxy-icosanoyl-coenzyme A.

Dans cet exemple, on suit le même mode opératoire que dans l'exemple 45 en utilisant comme réactif d'acylation l'ester de l'acide 4-fluoro-3-hydroxy-icosanoïque et du N-hydroxysuccinimide.

Le réactif d'acylation, les conditions réactionnelles et le résultat obtenu sont donnés dans le Tableau 7.

**Tableau 7**

| Exemple | Réactif d'acylation | $R^1$ | Conditions réactionnelles | Acyl-CoA | Rendement en produit isolé par CLHP |
|---|---|---|---|---|---|
| 45 | 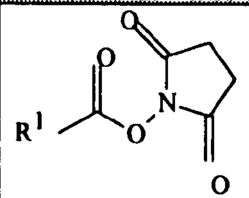 | $C_{17}H_{35}$ | THF<br>CsF/DCH-18-C-6<br>5 h | 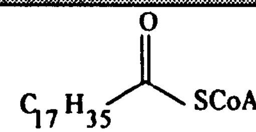 | 60 % |
| 46 | 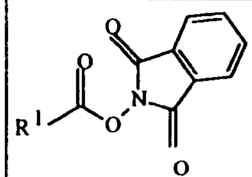 | $C_{17}H_{35}$ | THF<br>RbF/DCH-18-C-6<br>5 h | 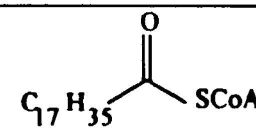 | 85 % |
| 47 | 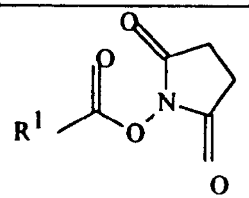 | $C_{19}H_{39}$ | THF<br>CsF/DCH-18-C-6<br>5 h | 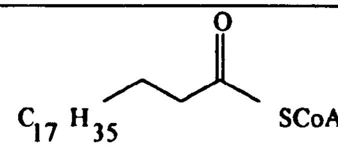 | 80 % |

**Tableau 7 (suite)**

| | | | | | |
|---|---|---|---|---|---|
| 48 | 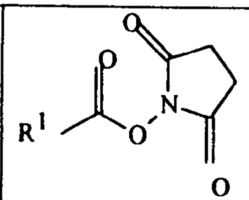 | $C_{19}H_{39}$ | THF<br>CsF/TDA-1<br>5 h | 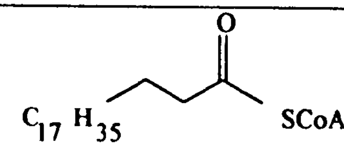 | 80 % |
| 49 | 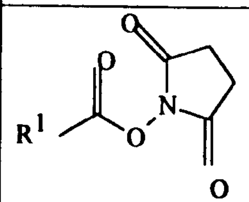 | $C_{19}H_{39}$ | THF<br>RbF/DCH-18-C-6<br>5 h | 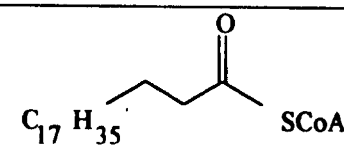 | 80 % |
| 50 | 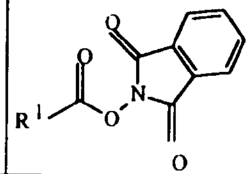 | $C_{19}H_{39}$ | THF<br>RbF/TDA-1<br>5 h | 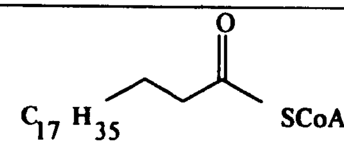 | 80 % |
| 51 | 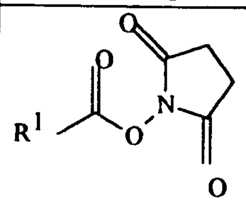 | 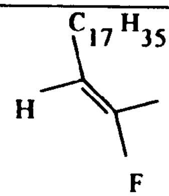 | THF<br>CsF/DCH-18-C-6<br>5 h | 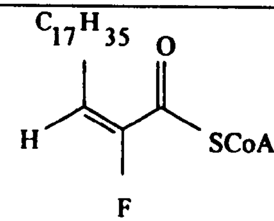 | 60 % |

**Tableau 7 (suite)**

| | | | | | |
|---|---|---|---|---|---|
| 52 | 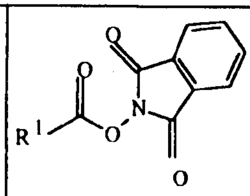 | 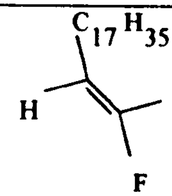 | THF<br>RbF/DCH-18-C-6<br>5 h | 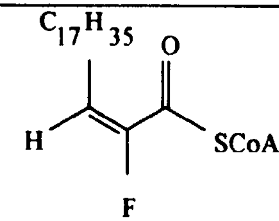 | 70 % |
| 53 | 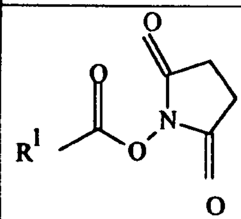 | 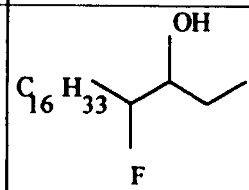 | THF<br>CsF/DCH-18-C-6<br>5 h | 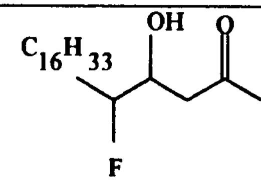 | 80 % |

THF : Tétrahydrofurane

DCH-18-C-6 : dicyclohexyl-18-C-6

TDA-1 : $N(CH_2CH_2OCH_2CH_2OCH_3)_3$

**Revendications**

1. Procédé de synthèse d'un dérivé acylé d'un thiol transporteur d'acides gras par réaction du thiol avec

un réactif d'acylation, caractérisé en ce qu'il comprend les étapes suivantes :

1°) préparer un thiol silylé ou stannylé en faisant réagir le thiol avec un réactif de silylation ou de stannylation pour remplacer au moins en partie les hydrogènes labiles du thiol par des groupes de formules $SiR_3$ ou $SnR_3$ dans lesquelles les R qui peuvent être identiques ou différents, sont des groupes alkyle ou aryle, et

2°) faire réagir dans un solvant organique anhydre le thiol silylé ou stannylé avec a) un réactif de déprotection pour éliminer les groupes $SiR_3$ ou $SnR_3$ et avec b) le réactif d'acylation pour obtenir le dérivé acylé du thiol.

**2.** Procédé selon la revendication 1, caractérisé en ce que le thiol est le coenzyme A.

**3.** Procédé selon l'une quelconque des revendications 1 et 2, caractérisé en ce que l'on prépare un thiol silylé dans la première étape.

**4.** Procédé selon la revendication 3, caractérisé en ce que le réactif de silylation est le 1-méthoxy-2-méthyl-1-triméthylsiloxypropène.

**5.** Procédé selon l'une quelconque des revendications 1 à 4, caractérisé en ce que l'on utilise dans la première étape un solvant organique constitué par l'acétonitrile ou le dichlorométhane.

**6.** Procédé selon l'une quelconque des revendications 3 à 5, caractérisé en ce que dans la première étape, on sépare le thiol silylé du milieu réactionnel par évaporation du solvant organique éventuel, de l'excès de réactif de silylation et des sous-produits de la réaction de silylation.

**7.** Procédé selon l'une quelconque des revendications 1 à 4, caractérisé en ce que le réactif de déprotection utilisé dans la deuxième étape est du fluorure de césium ou du fluorure de rubidium.

**8.** Procédé selon la revendication 7, caractérisé en ce que l'on fait réagir le fluorure de césium ou le fluorure de rubidium en présence d'un catalyseur constitué par un éther-couronne ou une amine.

**9.** Procédé selon la revendication 8, caractérisé en ce que l'éther-couronne est le dicyclohexyl-18-C-6.

**10.** Procédé selon la revendication 8, caractérisé en ce que l'amine est la tris(3,6-dioxaheptyl)amine.

**11** Procédé selon l'une quelconque des revendications 1 à 10, caractérisé en ce que le réactif d'acylation est un ester ou un anhydride mixte d'un acide carboxylique saturé ou insaturé.

**12.** Procédé selon la revendication 11, caractérisé en ce que le réactif d'acylation est un ester d'un acide carboxylique et du N-hydroxysuccinimide.

**13.** Procédé selon la revendication 11, caractérisé en ce que le réactif d'acylation est un ester d'un acide carboxylique et du N-hydroxyphtalimide.

**14.** Procédé selon l'une quelconque des revendications 11 à 13, caractérisé en ce que l'acide carboxylique est l'acide icosanoïque, l'acide octadécanoïque, l'acide 2-fluoro-2-icosénoïque ou l'acide 4-fluoro-3-hydroxy-icosanoïque.

**15.** Procédé selon l'une quelconque des revendications 1 à 14, caractérisé en ce que le solvant organique utilisé dans la deuxième étape est choisi parmi le diméthyl éther, le tétrahydrofurane (THF), l'acétonitrile et leurs mélanges.

**16.** Procédé selon l'une quelconque des revendications 1 à 15, caractérisé en ce que dans la deuxième étape, le rapport molaire du réactif d'acylation au co-enzyme A est de 1:1 à 2:1.

**17.** Procédé selon l'une quelconque des revendications 1 à 16, caractérisé en ce que l'on réalise la deuxième étape à une température de 0 à 25°C pendant une durée allant de 1h à 10h.

**18.** Coenzyme A silylé ou stannylé répondant à la formule suivante :

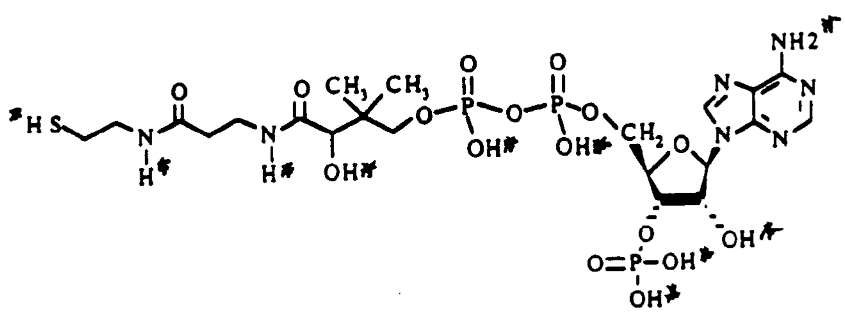

dans laquelle au moins une partie des hydrogènes labiles H* sont remplacés par un groupe de formule $SiR_3$ ou $SnR_3$ dans laquelle les R qui peuvent être identiques ou différents, sont des groupes alkyle ou aryle.

**19.** Coenzyme A silylé selon la revendication 18, caractérisé en ce que les R du groupe de formule $SiR_3$ représentent un groupe alkyle en $C_1$ à $C_4$.

**20.** Acyl-coenzymes A dans lesquels le groupe acyle est le groupe 4-fluoro 3-hydroxy-icosanoyle ou le groupe 2-fluoro-2-icosénoyle.

Office européen des brevets

# RAPPORT DE RECHERCHE EUROPEENNE

Numero de la demande
EP 94 40 0675

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| A | CHEMICAL ABSTRACTS, vol. 109, no. 13, 1988, Columbus, Ohio, US; abstract no. 106618s, T.SAITOH ET AL. 'Inhibitory Effect of Very-long-chain Monounsaturated Fatty-acyl-CoAs on the ELongation of Long-chain Fatty Acid in Swine Cerebral Microsomes.' page 260 ;colonne 1 ; * abrégé * & BIOCHIN. BIOPHYS. ACTA vol. 960, no. 3 , 1988 page 410-416 --- | 18,20 | C07H19/20 |
| A | CHEMICAL ABSTRACTS, vol. 112, no. 21, 1990, Columbus, Ohio, US; abstract no. 195482v, A.H.VAZ ET AL. 'Sex Pheromone Biosynthesis in the Housefly: Evidence for the Regulation of the Fatty Acyl-CoA Desaturation and Elongation System by 20-Hydroxyecdysone.' page 433 ;colonne 2 ; * abrégé * & ARCH. INSECT. BIOCHEM. PHYSIOL. vol. 12, no. 3 , 1989 pages 173 - 186 --- | 18,20 | DOMAINES TECHNIQUES RECHERCHES (Int.Cl.5)<br>C07H |

-/--

Le présent rapport a été établi pour toutes les revendications

| Lieu de la recherche | Date d'achèvement de la recherche | Examinateur |
|---|---|---|
| LA HAYE | 30 Mai 1994 | Scott, J |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

Office européen des brevets

# RAPPORT DE RECHERCHE EUROPEENNE

Numero de la demande
EP 94 40 0675

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| A | CHEMICAL ABSTRACTS, vol. 109, no. 9, 1988, Columbus, Ohio, US; abstract no. 68917d, T.SAITOH ET AL. 'Activation of Synthesis of Hexacosenoic Acid by Sulfhydryl Reagents in Swine Cerebral Microsomes.' page 271 ;colonne 2 ; * abrégé * & BIOCHEM. INT. vol. 16, no. 4 , 1988 pages 671 - 678 ----- | 18,20 | |
| | | | DOMAINES TECHNIQUES RECHERCHES (Int.Cl.5) |

Le présent rapport a été établi pour toutes les revendications

| Lieu de la recherche | Date d'achèvement de la recherche | Examinateur |
|---|---|---|
| LA HAYE | 30 Mai 1994 | Scott, J |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)